Europäisches Patentamt

European Patent Office

Office européen des brevets

(19)

(11) **EP 0 931 508 A1**

(12) **DEMANDE DE BREVET EUROPEEN**

(43) Date de publication:
28.07.1999 Bulletin 1999/30

(51) Int Cl.⁶: **A61B 8/06**

(21) Numéro de dépôt: 99200110.7

(22) Date de dépôt: 15.01.1999

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: 23.01.1998 EP 98400145
07.07.1998 FR 9808695

(71) Demandeur: **Koninklijke Philips Electronics N.V.**
**5621 BA Eindhoven (NL)**

(72) Inventeurs:
• **Cohen-Bacrie, Claude**
**75008 Paris (FR)**
• **Bruni, Fabrice**
**75008 Paris (FR)**
• **Bonnefous, Odile**
**75008 Paris (FR)**

(74) Mandataire: **Lottin, Claudine**
**Société Civile S.P.I.D.**
**156, Boulevard Haussmann**
**75008 Paris (FR)**

(54) **Procédé et échographe pour la détermination de la viscosité et du gradient de pression dans un vaisseau sanguin**

(57) Procédé de détermination de la viscosité et du gradient de pression dans un vaisseau sanguin, comprenant l'acquisition de n≥2 valeurs de vitesses du sang en correspondance avec un même nombre n de rayons du vaisseau sanguin, déterminés le long d'un diamètre situé à une position axiale donnée, formation d'un vecteur de vitesses du sang au moyen de ces n valeurs de vitesses du sang, et évaluation de ladite viscosité et du gradient de pression à partir d'une transformation dudit vecteur de vitesses du sang avec formation d'une relation linéaire liant directement un vecteur des débits (y) au vecteur des dérivées de vitesses (h) factorisé par la viscosité (μ) et au vecteur de gradient de pression (δ), et évaluation simultanée des deux valeurs à déterminer de viscosité (μ) et de gradient de pression (δ) à partir de cette équation directe. Les vitesses sont favorablement déterminées par corrélation de signaux ultrasonores fournis par un échographe.

Application : Imagerie médicale, échographie médicale

FIG.6

## Description

L'invention concerne un procédé de détermination de la viscosité et du gradient de pression dans un vaisseau sanguin. L'invention trouve son application dans le domaine de fabrication des appareils échographiques à ultrasons, appelés échographes, pour l'aide au diagnostic médical.

Dans le domaine médical, lorsqu'un patient a un accident vasculaire cérébral ou cardiaque, il est nécessaire de contrôler la viscosité du sang, car ce paramètre joue un rôle important dans le diagnostic de l'accident vasculaire et dans la définition d'un traitement ultérieur approprié, par exemple un traitement à base de médicaments fluidifiants. Actuellement, pour connaître la viscosité du sang, il est nécessaire de réaliser des mesures sur des échantillons sanguins obtenus par des prélèvements sur le patient. Il en résulte des inconvénients importants. Le résultat des mesures n'est pas immédiat, les prélèvements eux-mêmes sont invasifs donc potentiellement contaminants. Un autre inconvénient est que les prélèvements doivent être répétés périodiquement pour contrôler l'effet du traitement ce qui risque d'endommager les vaisseaux du patient. On ne connaît pas actuellement d'alternative à cette méthode de détermination de la viscosité du sang. Par moyen invasif on entend un prélèvement du sang qui nécessite par exemple l'introduction d'une aiguille dans le vaisseau sanguin pour emplir une seringue avec un échantillon de sang.

Il est déjà connu du brevet EP 0 430 374 (US 5 103 826, Bonnefous, Apr. 14, 1992), un appareil échographique à ultrasons muni d'un système de traitement de signaux ultrasonores pour déterminer des profils de la vitesse du sang dans une artère. L'appareil échographique comprend un transducteur émettant et recevant des signaux ultrasonores d'une région d'artère d'un patient, et un étage transmetteur connecté à la sonde. Le système de traitement estime la vitesse du sang dans l'artère, en fonction du temps et de la profondeur d'exploration, par un procédé de corrélation de signaux successifs renvoyés par le transducteur.

Un but de l'invention est de fournir un procédé apte à être mis en oeuvre, sans utiliser de moyens invasifs, pour permettre la détermination de la viscosité du sang s'écoulant dans une artère sous l'action de la pulsation cardiaque qui crée un gradient de pression, cette opération étant effectué avec une précision égale ou supérieure à celle qui était atteinte par la méthode connue incluant l'analyse de prélèvements.

Ce but est atteint par un procédé selon la revendication 1.

Ce procédé présente des avantages importants du fait qu'il est aussi précis que le procédé par prélèvements, qu'il est plus rapide et plus simple à mettre en oeuvre, et qu'il peut être répété un grand nombre de fois sans endommager les vaisseaux sanguins du patient, puisqu'il n'est pas invasif. Un autre avantage important réside dans le fait qu'aucun échantillon de sang n'étant prélevé ou traité, tout problème de contamination du patient ou du personnel de laboratoire est évité.

Un appareil échographique à ultrasons selon la revendication 10 est muni de moyens pour mettre en oeuvre ce procédé.

Le procédé est décrit ci-après en détail, en référence aux figures schématiques annexées dont :

la FIG. 1 qui représente sous forme de blocs fonctionnels, un diagramme de déroulement des étapes du procédé de détermination de la viscosité du sang dans une artère et du gradient de pression du à la pulsation cardiaque ;
la FIG.2A qui représente un axe d'exploration échographique (Z) vis-à-vis de l'axe longitudinal (X) d'un vaisseau sanguin ; la FIG.2B qui illustre le décalage temporel entre deux tirs échographiques et la FIG.2C qui illustre une fenêtre de résolution;
les FIGS.3A et 3B qui illustrent des premier et second signaux temporels et la FIG.3C qui illustre un signal de corrélation ;
la FIG.4 qui illustre un signal de corrélation en 1 bit;
la FIG.5 qui illustre un profil de vitesse le long d'un axe de tir échographique Z ;
la FIG.6 qui représente sous forme de blocs fonctionnels un diagramme de déroulement du procédé de transformation d'un vecteur de vitesses pour fournir les valeurs de viscosité et gradient de pression dans le vaisseau sanguin.

L'invention concerne un appareil d'échographie ultrasonore muni d'un système de traitement des signaux ultrasonores pour la détermination des paramètres physiologiques du sang s'écoulant dans une artère examinée in vivo dans une région tissulaire, et en particulier pour la détermination de la viscosité du sang de manière non invasive et en temps réel, et pour la détermination du gradient de pression sanguine dans cette artère.

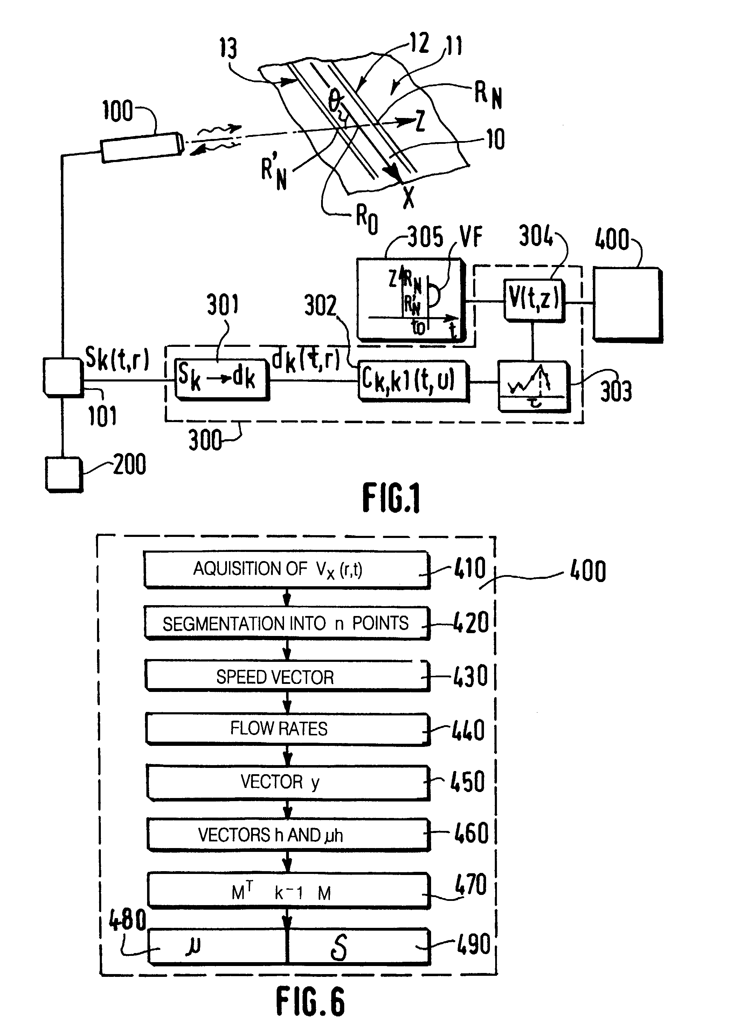

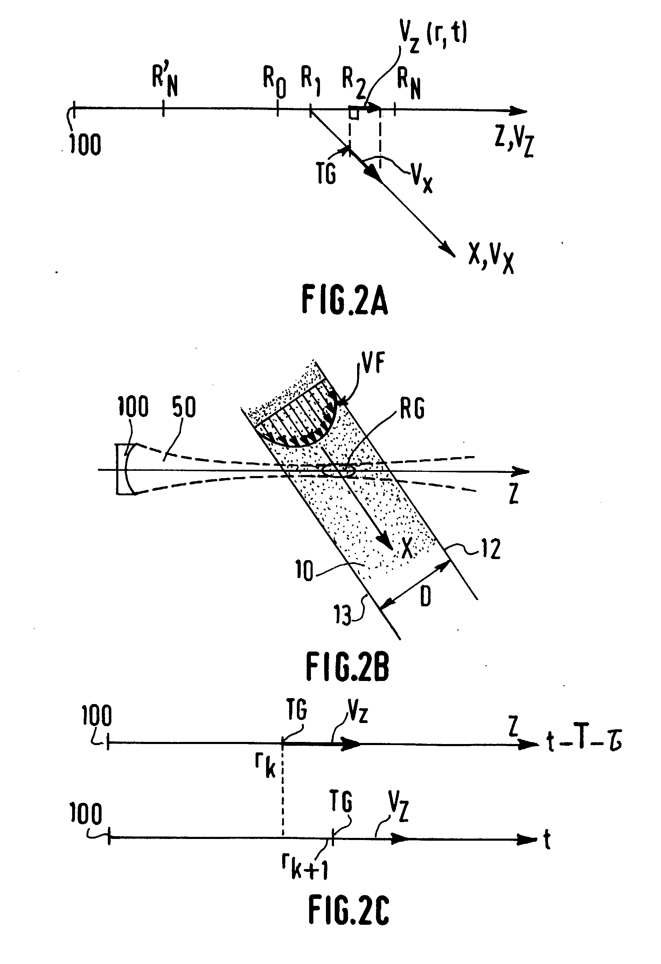

En référence à la FIG.1 et aux FIGS.2A, B, C, cet appareil comprend au moins un transducteur ultrasonore 100 associé à un étage d'émission 200 et à un étage de traitement numérique 300. Le transducteur 100 est appliqué à la région tissulaire 11 pour l'examen de l'artère 10 ayant des parois 12, 13. Le faisceau émis par le transducteur ultrasonore est de préférence large bande.

L'étage d'émission 200 comprend un séquenceur composé d'un oscillateur et d'un diviseur de fréquence qui commande, avec une période de récurrence T, un générateur dont les signaux électriques d'excitation sont envoyés vers le transducteur 100 qui les convertit en signaux temporels ultrasonores, qui sont émis avec la période de récurrence T, selon un axe de tir Z. Dans ce système d'émission, chaque signal temporel ultrasonore a un rang d'émission dans le temps noté k, k+1,..,de période T. L'étage d'émission 200 relié au transducteur 100 assure ainsi la formation d'un faisceau 50 ultrasonore pour l'exploration de la région tissulaire 11 incluant l'artère 10. Dans cet examen, on suppose que l'écoulement sanguin se fait selon l'axe longitudinal X de l'artère 10, et que l'axe de tir Z du faisceau ultrasonore à partir du transducteur 100 fait un angle $\Theta$ avec l'axe X. Les profondeurs d'exploration sont discrétisées sur l'axe de tir Z, en des points notés $R_0$, $R_1$.... $R_N$ repérés à partir d'un point 0, de coordonnée spatiale sur Z notée $r_o$, situé à l'intersection des axes X et Z à l'intérieur de l'artère, jusqu'à un point $R_N$ de coordonnée spatiale sur Z notée $r_N$ et situé sur une paroi par exemple 12 de l'artère 10.

En référence à la FIG.1, un étage séparateur 101 est inséré entre les étages 200, 300 et le transducteur 100 pour éviter l'aveuglement des étages 200 et 300 par les signaux d'émission. L'étage de traitement 300 comprend en sortie de l'étage séparateur 101 un amplificateur haute fréquence incluant une compensation de gain en fonction de la profondeur d'exploration dans la région tissulaire examinée. L'étage de traitement 300 comprend aussi un éliminateur 301 d'échos fixes qui fournit, à partir d'un signal $S_k(t,r)$ provenant de l'étage séparateur 101, un signal échographique temporel $d_k(r,t)$ débarrassé des composantes fixes dues notamment aux réflexions spéculaires sur les parois 12, 13 de l'artère 10 examinée.

Le signal $d_k(r,t)$ issu de l'éliminateur d'échos fixes 301 est traité de préférence selon une méthode de corrélation décrite dans le brevet EP 0 225 667 (US 4 803 390, Bonnefous). Cette méthode est brièvement rappelée ci-après.

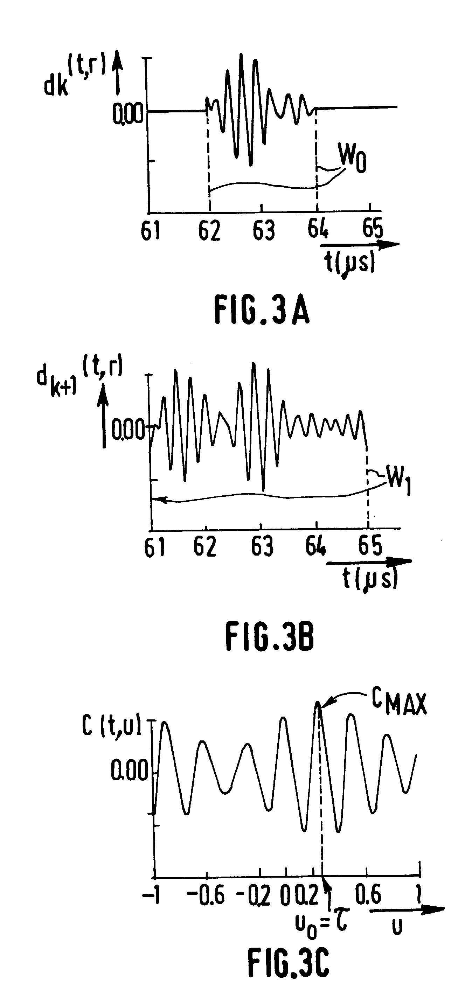

Selon cette méthode, le système de traitement 300 détermine la vitesse d'écoulement de structures du sang, par exemple des amas de globules rouges, appelées cibles, notées TG (en anglais : Target), à l'instant t où ces cibles coïncident avec l'axe de tir Z et en des points discrétisés sur l'axe de tir Z appartenant à l'intérieur de l'artère. Il est suffisant d'estimer les vitesses entre le centre 0 et le point $R_N$ sur la paroi 12 car les vitesses entre le centre 0 et le point $R'_N$ à l'intersection de l'axe Z et de l'autre paroi 13 de l'artère sont supposées symétriques. Les profondeurs d'exploration le long de l'axe Z sont données par des coordonnées spatiales r. Le temps où un écho est renvoyé par une cible située à une profondeur r est noté t. En particulier, le système de traitement 300 traite les signaux échographiques temporels de rang k notés $d_k (r,t)$ formés des intensités des échos renvoyés par les cibles en chaque point discrétisé de l'axe Z de profondeur $r_0$ à $r_N$ dans l'artère 10, et fournit la vitesse d'écoulement du sang en ces points. Les FIGs. 3A et 38 représentent de tels signaux temporels échographiques $d_k(r,t)$ et $d_{k+1}(r,t)$ avec en ordonnée l'intensité du signal échographique en unité arbitraire et en abscisse des coordonnées temporelles t en microsecondes qui correspondent aux coordonnées spatiales r le long de l'axe de tir Z.

Les FIGs.2A et 2B illustrent que l'appareil échographique n'est pas sensible aux vitesses autres que celles qui se rapportent à l'axe de tir Z. Donc, on ne considère ni les distances qui sont en réalité parcourues par les cibles TG selon l'axe X, ni les vitesses de ces cibles selon cet axe X, mais seulement leurs projections sur l'axe Z. La vitesse d'une cible est donc mesurée par sa composante $V_Z(r,t)$ le long de l'axe Z en fonction du temps t et de la profondeur d'exploration r. Sur la FIG.2C, on a noté $r_k$ et $r_{k+1}$ les profondeurs d'une cible TG renvoyant des échos dans les signaux de rang k et k+1 respectivement. La composante selon l'axe longitudinal X se déduit de $V_Z(r,t)$ par la relation :

$$V_X(r,t) = V_Z(r,t) /\cos\Theta \qquad (1)$$

Le système de traitement 300 met en oeuvre une technique de corrélation de deux signaux temporels échographiques successifs notés de rang k et k+1. La technique de corrélation demande, en référence à la FIG.2C, qu'un fort pourcentage de cibles présent dans le faisceau ultrasonore lors d'un premier tir de rang k reste présent dans le faisceau échographique lors d'un tir ultérieur de rang k+1, de manière à ce que la corrélation entre les deux signaux temporels successifs soit suffisamment forte. C'est pourquoi, en référence à la FIG.2B, la corrélation est effectuée dans une certaine fenêtre de résolution notée RG de dimension w (en anglais : Range Gate) choisie pour que cette demande soit facilement satisfaite. Par exemple, si le sang a une vitesse de flux de l'ordre de 1 m/s, si la période T entre deux excitations est égale à 100 μs ce qui correspond à une fréquence d'excitation de l'ordre de 10 kHz, le déplacement des cibles entre deux tirs successifs est de l'ordre de 0,1 mm. Ce déplacement est substantiellement petit devant les dimensions d'une cellule de résolution RG avantageusement choisies de l'ordre de 1 mm selon l'axe X et 0,3 mm selon l'axe Z. Dans ce cas, environ 20 % de cibles quittent la cellule de résolution pendant la période T, environ 20 % y entrent et un fort pourcentage de cibles reste présent.

L'idée basique du traitement mis en oeuvre par le système 300, pour la détermination de la vitesse en un point discrétisé de l'axe Z, est qu'un signal temporel ultrasonore $d_{k+1}(r,t)$ rétrodiffusé par une cible en mouvement, située à l'intérieur de la fenêtre de résolution RG, qui a été émis par un tir échographique selon Z, de rang k+1, au temps t, est la réplique d'un signal temporel antérieur émis par un tir échographique de rang k selon le même axe Z au temps t -T, rétrodiffusé par la même cible au temps t - T τ, où τ est le temps supplémentaire appelé décalage temporel (en anglais : Time Shift) mis par le signal temporel ultérieur pour parcourir le trajet sonde-cible-sonde, du fait du déplacement de la cible durant l'intervalle T.

Ainsi, au premier ordre, deux signaux temporels échographiques successifs rétrodiffusés par la même cible sont liés par la relation :

$$d_{k+1}(t) = d_k(t\text{-}T - \tau) \qquad (2)$$

D'où il résulte que le décalage temporel $\tau$ est donné par :

$$\tau = 2V_Z(r,t)/c \qquad (3)$$

où c est la vitesse du son dans le vide et par extension dans les tissus. La formule (3) permet de déterminer les composantes $V_Z(r,t)$ des vitesses des cibles en chaque point discrétisé d'ordonnées spatiales r en faisant une approximation basée sur le fait que la vitesse axiale $V_Z(r,t)$ est très petite devant la vitesse du son c, et que le déplacement d'une cible est très petit devant la distance du transducteur à la cible durant une période T.

Par conséquent, la vitesse du flux peut être mesurée par un appareil échographique ayant un système de traitement 300 munis de moyens pour mesurer le décalage temporel $\tau$ induit par les cibles entre deux tirs échographiques.

Au premier ordre, la fonction d'intercorrélation entre les deux signaux temporels successifs est de la forme :

$$C_{k,k+1}(t_0,u) = \int_{t_0}^{t_0+w_0} d_k(t) \; d_{k+1}(t+u) \, dt \qquad (4)$$

où le temps $t_0$ est lié à la profondeur d'exploration r par $t_0 = 2r/c$, $w_0$ est la largeur de la fenêtre de résolution sur l'axe du temps, et u est un paramètre de décalage temporel de la fonction d'intercorrélation (en anglais : cross-correlation).

En introduisant l'équation (2) dans l'équation (4), il vient :

$$C_{k,k+1}(t_o,u) = C_{k,k}(t_0,u - \tau) \qquad (5)$$

La fonction $C_{k,k}(t_o,u - \tau)$ est une fonction d'autocorrélation, et, ce fait est maximale pour $u = \tau$. Dans une hypothèse d'une situation idéale de vitesse axiale uniforme, le maximum de la fonction de corrélation correspond au décalage de temps $\tau$ et par conséquent permet une détermination de la vitesse du sang pour le tir échographique au rang k selon la relation :

$$V_Z(r,t) = (c/2) \; (\tau/T) \qquad (6)$$

En référence à la FIG.1, le circuit 302 d'intercorrelation du système de traitement 300 détermine les vitesses $V_Z(r,t)$ en cherchant pour quel paramètre u la fonction $C_{k,k+1}(t_0,u)$ est maximale. A cet effet, la fonction d'intercorrélation est échantillonnée à un pas d'échantillonnage $\Delta t$, de façon à obtenir 2m+1 valeurs de la fonction de corrélation, m étant un scalaire. La valeur maximale de ces 2m+1 valeurs correspondant à $u = u_0$ permet de mesurer $\tau$ en utilisant l'égalité : $\tau = u_0$ (7).

Selon cette méthode, il n'y a pas d'ambiguïté à la mesure des vitesses parce qu'il n'existe qu'un seul maximum à cette fonction de corrélation.

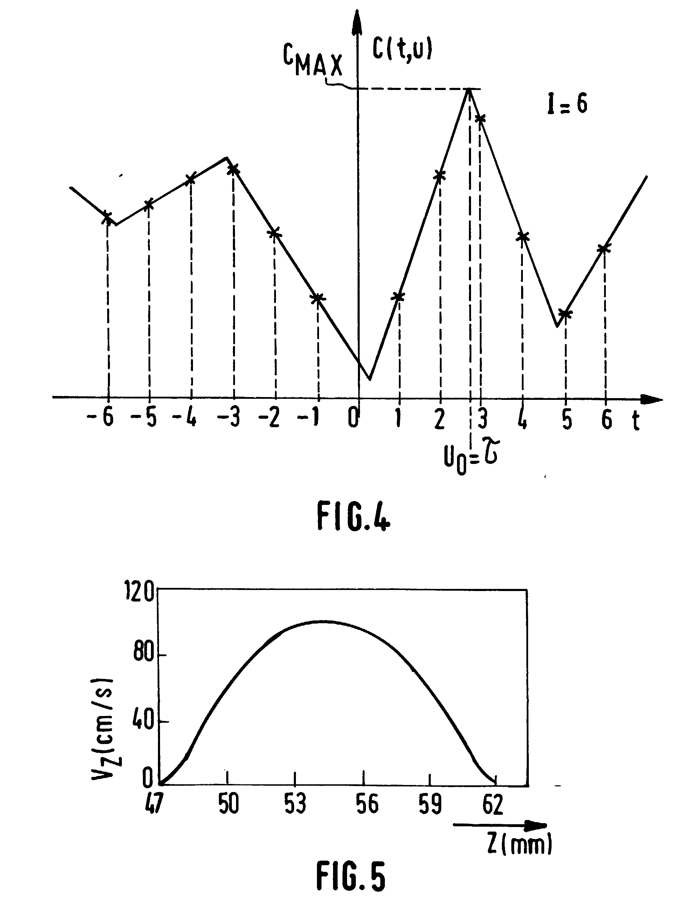

La méthode de décalage temporel dérive du déplacement réel des cibles entre deux tirs successifs dans le temps selon le même axe Z. C'est pourquoi la fenêtre de résolution doit être habilement choisie. La FIG.3A illustre une première fenêtre de résolution de dimension $w_0 = 2\,\mu S$ appliquée à un signal temporel $d_k(r,t)$ et la FIG.4B illustre une seconde fenêtre de résolution de dimension $w_1 = 4\,\mu s$. appliquée au signal ultérieur $d_{k+1}(r,t)$. La FIG.4C illustre la corrélation C des signaux temporels $d_k(r,t)$ et $d_{k+1}(r,t)$ qui conduit à la détermination d'un pic $C_{MAX}$ de corrélation pour $\tau$ de l'ordre de $0,3\,\mu s$ ce qui permet de calculer la vitesse $V_Z(r,t)$ par la relation (6).

Afin de s'affranchir des erreurs inhérentes à l'échantillonnage dans la détermination du maximum de la fonction de corrélation, le système de traitement 300 comprend favorablement un circuit 303 de multiplexage-interpolation fournissant à partir des valeurs des fonctions de corrélation une estimée plus précise de valeur du pic de corrélation et donc de la vitesse correspondante. Dans ce type de traitement, la corrélation entre signaux est une corrélation dite «1bit » en ce sens que les signaux $y_{k+1}(r,t)$ et $y_k(r,t)$ utilisés précédemment sont réduits à leur signe. On sait que, dans ce cas, le pic $C_{MAX}$ de la fonction de corrélation est de forme triangulaire isocèle. Comme illustré par la FIG.4, la connaissance de cette forme permet de n'effectuer le calcul de corrélation qu'en des points discrets des signaux temporels, et, une fois déterminés un certain nombre de points, permet à partir du point le plus haut et de ses deux voisins de reconstituer complètement, par interpolation linéaire, le pic de corrélation $C_{MAX}$ et donc de déterminer avec précision l'emplacement de $u_0 = \tau$, d'où l'on peut déduire $V_Z(r,t)$.

En référence à la FIG. 1, les valeurs trouvées pour la vitesse V(t,z) sont stockées dans une mémoire 304 de stockage en vue de traitements ultérieurs. Comme illustré par la FIG.5, en reportant les valeurs de vitesse $V_Z(r,t)$ en cm/s en ordonnée et les profondeurs r en mm en abscisse, on obtient un profil des vitesses sur l'axe Z dans l'artère au temps t. De même que l'axe Z est discrétisé en N points d'une part de O et en N points d'autre part de O, le profil des vitesses est discrétisé avec le même nombre de points.

En référence à la FIG.1, à l'aide de moyens de visualisation connus, il est possible d'afficher sur un écran 305 une image en couleur de l'écoulement du sang qui est dérivée de l'évolution au cours du temps du profil de vitesse dans le vaisseau. Le codage couleur de la vitesse dans cette image est notée CVI (de l'anglais ; Color Velocity Imaging).

Un avantage important de la méthode échographique de corrélation en 1 bit est que les profils des vitesses sont obtenus en temps réel. Cependant toute autre méthode apte à fournir des données de vitesse du sang dans un vaisseau avec une précision suffisante peut être utilisée pour mener à bien l'évaluation de la viscosité et du gradient de pression comme décrit ci-après.

Les profils de vitesse du sang, à la même localisation Z, varient dans le temps. Les caractéristiques d'un fluide telles que la viscosité, la compressibilité, l'isotropie ; la nature du flux telle que laminaire ou turbulente ; les propriétés du tube qui contient le fluide, telles que la rigidité, l'élasticité, ou la viscoélasticité ; et la forme du gradient de pression telle que large-bande ou bande étroite, sont des paramètres qui dictent la forme des profils de vitesse.

On suppose ci-après que le sang dans une artère est assimilable à un fluide Newtonien ayant un flux laminaire s'écoulant dans un tube rigide. Comme illustré par la FIG.5, sous un gradient de pression noté $dP/dx$, la vitesse V(r, t) suit une loi de Navier-Stokes, exprimée par la formule connue (8) :

$$-\frac{dP}{dx} = \rho\left(\frac{dV}{dt}\right) - \mu\left(\frac{d^2V}{dr^2}\right) + \frac{1}{r}\frac{dV}{dr} \tag{8}$$

où $\rho$ est la densité du fluide et où $\mu$ est la viscosité

La loi de Navier-Stokes, qui lie la viscosité et le gradient de pression à la vitesse, ne permet cependant pas de calculer directement la viscosité du fluide et le gradient de pression en fonction de la vitesse du fluide. Le calcul de la viscosité du fluide par inversion de l'équation de Navier-Stokes conduit à des expressions analytiques complexes.

Selon l'invention, ces difficultés sont évitées, et la viscosité et le gradient de pression sont déterminés en temps réel par un procédé de traitement de données incluant une inversion de la relation (8) qui consiste à estimer la viscosité et le gradient de pression à partir des données fournies par des profils de vitesse préalablement déterminés de préférence comme décrit précédemment.

Sous l'hypothèse d'un flux laminaire, l'équation de Navier-Stokes gouverne les variations de la vitesse V(r,t) pour r et t donnés. Pour les points discrétisés $R_J$ de $R_0$ à $R_N$, de coordonnées spatiales $r_J$ de $r_0$ à $r_N$ sur l'axe Z, l'intégration de l'équation (8) de Navier-Stokes entre $r = r_1$ et $r = r_n$ fournit l'expression (9) suivante, la dérivée radiale de $V_X$ étant nulle :

$$\frac{\rho}{\pi r_j}\frac{dQ(r_j)}{dt} = -\frac{\partial P}{\partial x} - \mu\frac{2}{R}\frac{\partial V_X(r_j)}{\partial r} \tag{9}$$

où $r_j$ est la profondeur d'exploration

Chaque membre de l'équation (10) est une fonction du temps t et du rayon $r_j$. Pour chaque échantillon de temps, la relation peut être écrite sous une forme vectorielle, chaque vecteur étant formé par les valeurs des différentes quantités à différentes profondeurs d'exploration $r_j$. D'où :

$$y = \begin{bmatrix} \dfrac{\rho}{\pi\, r_1^{\,2}} & \dfrac{dQ(r_1)}{dt} \\[2em] \dfrac{\rho}{\pi\, r_N^{\,2}} & \dfrac{dQ(r_n)}{dt} \end{bmatrix} \;\;;\;\; h = \begin{bmatrix} \dfrac{2}{r_1} & \dfrac{dV_X}{dr}(r_1) \\[2em] \dfrac{2}{r_n} & \dfrac{dV_X}{dr}(r_n) \end{bmatrix} \;\;;\;\; \delta = \delta \begin{bmatrix} 1 \\ 1 \\ \\ 1 \end{bmatrix} = -\dfrac{\partial P}{\partial x} \begin{bmatrix} 1 \\ 1 \\ \\ 1 \end{bmatrix} \quad (10)$$

L'équation du problème direct peut donc être mise sous la forme :

$$y = \mu h + \delta + b \tag{11}$$

où b est un bruit blanc gaussien qui modélise les erreurs de mesure. En accord avec l'équation (11), y est un vecteur gaussien dont la moyenne est égale à :

$$\mu h + \delta \tag{12}$$

La phase essentielle du procédé consiste à inverser cette équation qui modélise le problème direct (11). La solution du problème inverse est obtenue de préférence au moyen d'un estimateur de maximum de vraisemblance connu (Maximum Likelihood Estimator) utilisé pour estimer à la fois la viscosité $\mu$ et le gradient de pression $\delta$.

On cherche les valeurs $\mu$ et $\delta$ qui maximisent la vraisemblance de y connaissant $\mu$ et $\delta$ données par une formule (13) :

$$(\hat{\mu}, \hat{\delta}) = \arg\max{}_{(\mu,\delta)}\left[ p(y|\mu,\delta) \right] \qquad (13)$$

où $p(y|\mu, \delta)$ est la probabilité de y connaissant $\mu$, $\delta$, et où $\hat{\mu}$ et $\hat{\delta}$ sont des estimateurs de $\mu$ et $\delta$ appelées estimateurs du maximum de vraisemblance. Ceci est équivalent à rechercher le minimum d'une fonction de coût notée $W(\mu,\delta)$:

$$W(\mu,\delta) = (y - \mu h - \delta)^T\, K_n^{-1}\, (y - \mu h - \delta) \tag{14}$$

où $K^{-1}{}_n$ est une matrice diagonale dont les coefficients dépendent de la confiance que l'on accorde aux valeurs de y, ce qui conduit aux expressions suivantes de $\mu$ et $\delta$ :

$$\hat{\mu} = \frac{y^T K_n^{-1} h - \dfrac{h^T K_n^{-1} 1\, y^T K_n^{-1} 1}{1^T K_n^{-1} 1}}{h^T k_n^{-1} h - \dfrac{h^T K_n^{-1} 1\, h^T K_n^{-1} 1}{1^T k_n^{-1} 1}} \tag{16a}$$

$$\hat{\delta} = \frac{h^T K_n^{-1} y - \hat{\mu} h^T K_n^{-1} h}{h^T K_n^{-1} 1} \tag{17a}$$

En première approximation, on suppose que le bruit est distribué de façon identique, de manière à ce que la matrice $K_n$ soit proportionnelle à la matrice identité. Ceci produit une première approximation de la solution :

$$\hat{\mu}(0) = \frac{y^T h - \dfrac{h^T 1\, y^T 1}{1^T 1}}{h^T h - \dfrac{h^T 1\, h^T 1}{1^T 1}} \tag{16b}$$

$$\hat{\delta}(0) = \frac{h^T y - \hat{\mu}\, h^T h}{h^T 1} \tag{17b}$$

Selon l'invention, on propose un procédé de détermination de la viscosité $\mu$ fondé sur cette méthode de résolution du problème inverse, à partir du problème direct constitué par la relation (11) et des profils de vitesse déterminés de préférence par échographie, ou par toute autre méthode donnant des résultats équivalents, en utilisant cet estimateur de maximum de vraisemblance selon des étapes décrites ci-après.

En référence aux FIG. 1 et 6, un processeur 400 est associé au système de traitement 300 de l'appareil échographique ou d'un système fournissant les vitesses. En échographie, le système de traitement 300 est «matériel » au sens HARDWARE utilisé par l'homme du métier, et le processeur 400 est programmé au sens SOFTWARE également connu. Le processeur 400 peut faire partie de l'échographe ou bien d'une station de travail couplée à l'échographe. La programmation du processeur 400 définit les différentes étapes nécessaires pour déterminer la viscosité $\mu$ et le gradient de pression $\delta$ selon le procédé de l'invention illustré par les blocs fonctionnels de la FIG.6. Ce procédé comprend des étapes de :

acquisition 410 des profils de vitesses $V_X(r,t)$ à partir des profils de vitesse $V_Z(r,t)$ fournies par le système de traitement 300, en utilisant la formule (1) ;

échantillonnage 420 d'un profil de vitesse discrétisé donné, en utilisant un nombre n de points $R_1$ à $R_n$ parmi les N points de discrétisation $R_1$ à $R_N$, tel que $2 \leq n \leq N$ ; les n points de segmentation définissent des rayons $r_1$ à $r_n$ du tube assimilé au vaisseau sanguin, notés de manière générale $r_j$;

détermination 430 d'un vecteur de vitesses constitué des vitesses aux points de segmentations $R_1$ à $R_n$, ces vitesses étant notées $V_X(r_1)$ à $V_X(rp)$ et d'une manière générale $V_X(r_j)$,

évaluation 440 des débits du fluide dans le tube, c'est-à-dire du sang dans le vaisseau, dans les sections de tube, définies par les rayons $r_j$ de $r_1$ à $r_n$, ce qui fournit respectivement les débits notés $Q(r_j)$ de $Q(r_1)$ à $Q(r_n)$ ;

calcul 450 du vecteur y selon la formulation (10) en effectuant des calculs des quantités

$$\frac{\rho}{\pi r_j} \frac{dQ(r_j)}{dt} \tag{20}$$

pour $r_j$ de $r_1$ à $r_n$ ;

Ces calculs (20a) comprennent des sous-étapes de :
détermination du débit $Q(r_j)$ calculé selon la formulation suivante :

$$Q(r_j) = \sum_{i=1}^{i=j} 2\pi\, r_i\, V_X(r_i) \qquad (20a),$$

détermination des dérivées temporelles des débits $Q(r_j)$ selon :

$$\frac{dQ(r_j)}{dt} \text{ pour } r_j \text{ de } r_1 \text{ à } r_n \tag{20b},$$

et multiplication des dérivées temporelles des débits par les quantités respectives avec $r_j$ de $r_1$ à $r_n$ :

$$\rho/\pi\, r_j \qquad\qquad (20c)$$

où $\rho$ est la masse volumique du fluide supposée environ égale à 1 ;

calcul 460 du vecteur h selon la formulation (10) en effectuant des calculs des quantités :

$$\frac{2}{r_j}\frac{\partial V_x(r_j)}{\partial r} \qquad\qquad (30)$$

qui sont les dérivées axiales des vitesses pour chaque point $R_j$ de $R_1$ à $R_N$ définissant les rayons $r_j$ ;
calcul 470 d'un produit matriciel : $M^T K^{-1} M$, où la matrice M est donnée par la formule : $M= y - \mu h - \delta$, et où $M^T$ est la matrice transpose de M.
calcul 480 de la viscosité $\mu$ selon les formulations (16)
calcul 490 du gradient de pression $\delta = \frac{dp}{dx}$ selon (17)

Pour effectuer les étapes 410 à 490, le processeur est programmé par les formules de dérivées temporelles de débit, de dérivées axiales de vitesse, et de calcul matriciel. Le système de traitement 300 fournit automatiquement en temps réel les profils de vitesse et le processeur 400 fournit automatiquement subséquemment la viscosité et le gradient de pression dans un délai total extrêmement court c'est-à-dire en temps réel.

Grâce au procédé selon l'invention, pour résoudre l'équation (8) qui a deux inconnues $\mu$ et $\delta$, on procède donc par une inversion du problème direct qui utilise n points discrétisés d'un profil de vitesse pour résoudre en réalité n équations à deux inconnues, avec $n \geq 2$. Dans le cas de la détermination de la viscosité du sang, il est intéressant de sélectionner un nombre $n > 2$ de points de discrétisation pour disposer de plus de 2 équations indépendantes à deux inconnues pour la détermination des 2 inconnues. En effet, si l'on se réduit à $n = 2$, on peut se trouver occasionnellement devant une indétermination qui ne permet pas de déterminer les deux inconnues. Dans ces conditions les moyens de calculs sont bloqués. Alors que si l'on choisit favorablement un nombre $n>2$ de points de segmentation, il se trouve toujours un nombre suffisant d'équations pour déterminer les deux inconnues $\mu$ et $\delta$.

## Revendications

1. Procédé de détermination de la viscosité et du gradient de pression dans un vaisseau sanguin, comprenant des étapes de :

   acquisition d'une pluralité $n\geq 2$ de valeurs de vitesses du sang en correspondance avec un même nombre n de rayons du vaisseau sanguin, déterminés le long d'un diamètre situé à une position axiale donnée, et à différents instants,
   formation d'un vecteur de vitesses du sang au moyen de ladite pluralité de valeurs de vitesses du sang,
   évaluation de ladite viscosité et du gradient de pression à partir d'une transformation dudit vecteur de vitesses du sang.

2. Procédé selon la revendication 1, comprenant, pour effectuer la transformation dudit vecteur de vitesses du sang des étapes de :

   formation d'un vecteur des débits (y) constitué d'une même pluralité $n\geq 2$ de valeurs de débits du sang à travers des sections du vaisseau sanguin en correspondance avec les n valeurs de rayons,
   formation d'un vecteur (h) des dérivées des vitesses constitué d'un quotient de la même pluralité $n\geq 2$ de dérivées radiales des vitesses par les rayons correspondants, ce vecteur étant factorisé par la viscosité ($\mu$) à déterminer,
   formation d'un vecteur de gradient de pression ($\delta$) constitué du produit du gradient de pression à déterminer par une matrice identité,
   formation d'une relation linéaire liant directement le vecteur des débits (y) au vecteur des dérivées de vitesses (h) factorisé par la viscosité ($\mu$) et au vecteur de gradient de pression ($\delta$),
   évaluation simultanée des deux valeurs à déterminer de viscosité ($\mu$) et de gradient de pression ($\delta$) à partir de cette équation directe.

3. Procédé selon la revendication 2, comprenant l'évaluation simultanée des deux valeurs à déterminer de viscosité ($\mu$) et de gradient de pression ($\delta$) par une opération d'inversion de la relation linéaire directe liant le vecteur des débits (y) au vecteur des dérivées de vitesse (h) factorisé par la viscosité ($\mu$) et au vecteur gradient de pression ($\delta$).

4. Procédé selon la revendication 3, comprenant, pour effectuer l'inversion de la relation linéaire directe liant le vecteur des débits (y) au vecteur des dérivées de vitesse (h) factorisé par la viscosité ($\mu$) et au vecteur gradient de pression ($\delta$), une étape d'estimation du maximum de vraisemblance du vecteur des débits (y) connaissant la viscosité ($\mu$) et le gradient de pression ($\delta$).

5. Procédé selon la revendication 4, comprenant, pour estimer ledit maximum de vraisemblance du vecteur des débits (y) connaissant la viscosité ($\mu$) et le gradient de pression ($\delta$) des étapes de :

   évaluation à partir de ladite relation linéaire directe d'une première matrice (M) telle que:

   $$M = y - \mu h - \delta$$

   et d'une seconde matrice transpose de la première matrice ($M^T$),
   évaluation d'une troisième matrice (K) qui est une matrice diagonale dont les coefficients dépendent de la distribution du bruit,
   et évaluation dudit maximum de vraisemblance du vecteur des débits (y) connaissant la viscosité ($\mu$) et le gradient de pression ($\delta$) en effectuant une recherche du minimum d'une fonction de coût (W) qui est le produit de la première, troisième et seconde matrices tel que :

   $$M K^{-1} M^T$$

6. Procédé selon la revendication 5, où la troisième matrice diagonale (K) est une matrice identité diagonale, le bruit étant considéré uniformément réparti.

7. Procédé selon la revendication 2, où le nombre des valeurs de vitesses est deux (n=2) et où l'évaluation des deux valeurs à déterminer de viscosité ($\mu$) et gradient de pression ($\delta$) comprend des étapes de :
   formation d'un couple de relations linéaires directes liant le vecteur des débits (y) au vecteur des dérivées de vitesse (h) factorisé par la viscosité ($\mu$) et au vecteur gradient de pression ($\delta$), de la forme

   $$y = \mu h + \delta$$

   résolution du couple de relations linéaires directes, au moyen des deux valeurs de débits et de deux valeurs de dérivées des vitesses, pour fournir la viscosité ($\mu$) et le gradient de pression ($\delta$).

8. Procédé selon l'une des revendications 1 à 7 comprenant pour l'acquisition de valeurs de vitesses des étapes de :

   acquisition de signaux temporels fournis par un transducteur ultrasonore explorant un vaisseau sanguin selon un axe de tir échographique (Z),
   traitement des signaux temporels dans le domaine temporel pour fournir des signaux corrélés temporellement et un profil de vitesses du sang discrétisé le long de la direction de l'axe de tir,
   estimation d'un vecteur de vitesses du sang à partir d'un nombre $n \geq 2$ de valeurs de vitesses sélectionnées sur le profil des vitesses à différentes profondeurs d'exploration (r) sur l'axe de tir (Z) dans le vaisseau sanguin.

9. Procédé selon la revendication 8, où l'étape de traitement des signaux échographiques temporels est une corrélation temporelle en 1 bit.

10. Appareil échographique pour la mesure et l'affichage de paramètres physiologiques du sang s'écoulant dans un vaisseau sanguin, incluant des moyens de mesure de la viscosité et du gradient de pression du sang, ces moyens comprenant :

    des moyens (200) pour l'acquisition de signaux temporels fournis par un transducteur (100) ultrasonore explorant un vaisseau sanguin selon un axe de tir échographique (Z),
des moyens (300) de traitement des signaux temporels dans le domaine temporel pour fournir des signaux corrélés temporellement et un profil de vitesses discrétisé le long de la direction de l'axe de tir (Z),
des moyens (400) pour :
l'estimation d'un vecteur de vitesse du sang à partir d'un nombre $n \geq 2$ de valeurs de vitesses sélectionnées sur le profil des vitesses à différentes profondeurs d'exploration (r) sur l'axe de tir (Z) dans le vaisseau sanguin, et l'évaluation de la viscosité du sang ($\mu$) et du gradient de pression ($\delta$), par une transformation dudit vecteur des vitesses en utilisant un procédé selon l'une des revendications 2 à 9.

**FIG.1**

AQUISITION OF $V_x (r,t)$ — 410

400

SEGMENTATION INTO n POINTS — 420

SPEED VECTOR — 430

FLOW RATES — 440

VECTOR y — 450

VECTORS h AND μh — 460

$M^T$ $k^{-1}$ M — 470

480

μ

$S$ — 490

**FIG.6**

FIG.2A

FIG.2B

FIG.2C

FIG.3A

FIG.3B

FIG.3C

**FIG.4**

**FIG.5**

Office européen
des brevets

**RAPPORT DE RECHERCHE EUROPEENNE**

Numéro de la demande

EP 99 20 0110

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| Y | WO 95 12822 A (UNIV CALIFORNIA) 11 mai 1995 * page 4, ligne 14 - page 7, ligne 21; tableaux 1,2 * | 1-3,8-10 | A61B8/06 |
| Y | EP 0 603 967 A (PHILIPS ELECTRONIQUE LAB ;KONINKL PHILIPS ELECTRONICS NV (NL)) 29 juin 1994 * page 7, ligne 13 - page 15, ligne 39; tableaux 1-4 * | 1-3,8-10 | |
| A | KIRK SHUNG K ET AL: "THE EFFECTS OF HEMATOCRIT, SHEAR RATE, AND TURBULENCE ON ULTRASONIC DOPPLER SPECTRUM FROM BLOOD" IEEE TRANSACTIONS ON BIOMEDICAL ENGINEERING, vol. 39, no. 5, 1 mai 1992, pages 462-469, XP000294195 * page 463, colonne de gauche, ligne 47 - page 465, colonne de gauche, ligne 33; tableau 1 * | 1 | |

**DOMAINES TECHNIQUES RECHERCHES (Int.Cl.6)**

A61B

Le présent rapport a été établi pour toutes les revendications

| Lieu de la recherche | Date d'achèvement de la recherche | Examinateur |
|---|---|---|
| BERLIN | 16 avril 1999 | Weihs, J |

EPO FORM 1503 03.82 (P04C02)

**ANNEXE AU RAPPORT DE RECHERCHE EUROPEENNE
RELATIF A LA DEMANDE DE BREVET EUROPEEN NO.**          EP 99 20 0110

La présente annexe indique les membres de la famille de brevets relatifs aux documents brevets cités dans le rapport de
recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-04-1999

| Document brevet cité au rapport de recherche | | Date de publication | Membre(s) de la famille de brevet(s) | | Date de publication |
|---|---|---|---|---|---|
| WO 9512822 | A | 11-05-1995 | US | 5532593 A | 02-07-1996 |
| | | | AT | 169121 T | 15-08-1998 |
| | | | AU | 8097294 A | 23-05-1995 |
| | | | DE | 69412121 D | 03-09-1998 |
| | | | EP | 0727050 A | 21-08-1996 |
| EP 0603967 | A | 29-06-1994 | JP | 6237929 A | 30-08-1994 |
| | | | US | 5411028 A | 02-05-1995 |

EPO FORM P0460

Pour tout renseignement concernant cette annexe : voir Journal Officiel de l'Office européen des brevets, No.12/82